# EUROPEAN PATENT SPECIFICATION

(11) **EP 1 241 246 B1**
(45) Date of publication and mention of the grant of the patent: **10.12.2008**
(21) Application number: 00985850.7
(22) Date of filing: 22.12.2000
(51) Int. Cl.: C12N 1/20, C12N 1/21, C12N 9/12, C12P 13/04, C12P 13/06, C12P 13/08, C12P 13/14, C12P 13/18, C12R 1/13, C12R 1/15

(54) **PROCESS FOR PRODUCING L-AMINO ACID**
VERFAHREN ZUR HERSTELLUNG VON L-AMINOSÄURE
PROCEDE DE PRODUCTION D'ACIDE L-AMINE

(30) Priority: 24.12.1999 JP 36809699
(43) Date of publication of application: 18.09.2002
(73) Proprietor: Ajinomoto Co., Inc., Tokyo 104-8315 (JP)
(72) Inventor: SUGIMOTO, Masakazu, Ajinomoto Co. Inc., Fermentat., Kawasaki-shi, Kanagawa 210-8681 (JP); NAKAI, Yuta, Ajinomoto Co., Inc., Fermentation, Kawasaki-shi, Kanagawa 210-8681 (JP); ITO, Hisao, Ajinomoto Co., Inc., Fermentation, Kawasaki-shi, Kanagawa 210-8681 (JP); KURAHASHI, Osamu, Ajinomoto Co. Inc., Fermentation, Kawasaki-shi, Kanagawa 210-8681 (JP)
(74) Representative: Strehl Schübel-Hopf & Partner
(86) International application number: PCT/JP2000/009164
(87) International publication number: WO 2001/048146

(56) References cited:
- DATABASE EMBL [Online] fruA gene; 58.6% gapped identity SEQ ID: 13/14, 6 July 1989 (1989-07-06) "E. coli phosphostransferase system II (fruA) gene, complete cds." retrieved from EBI Database accession no. M23196 XP002268173 & PRIOR, T. ET AL. : "Nucleotide Sequence of fruA, the Gene Specifying Enzmye II fru of the Phosphoenolpyruvate-dependent Sugar Phosphotransferase System in Escherichia coli K12" J.GEN. MICROBIOL., vol. 134, 1998, pages 2757-2768,
- H. DOMINGUEZ ET AL.: 'Complete sucrose metabolism requires fructose phosphotransferase activity in corynebacterium glutamicum to ensure phosphorylation of liberated fructose' APPL. ENVIRON. MICROBIOL. vol. 62, no. 10, 1996, pages 3878 - 3880, XP002937887
- TREVORI PRIOR ET AL.: 'Nucleotide sequence of fruA, the gene specifying enzyme II(fru) of the phosphoenolpyruvate-dependent sugar phosphotransferase system in escherichia coli K12' J. GEN. MICROBIOL. vol. 134, 1998, pages 2757 - 2768, XP002937888
- K. JAHREIS ET AL.: 'Nucleotide sequence of the ilvH-fruR gene region of escherichia coli K12 and salmonella typhimurium LT2' MOL. GEN. GENET. vol. 226, no. 1-2, 1991, pages 332 - 336, XP002937889

### Sequence Listing

<110> Ajinomoto Co., Inc.
<120> Methods for Producing L-Amino Acods and Novel Gene
<130> EPA-53929
<150> JP 11-368096
   <151> 1999-12-24
<160> 14
<170> PatentIn Ver. 2.0
<210> 1
   <211> 24
   <212> DNA
<213> Artificial Sequence
<220>
   <223> Description of Artificial Sequence:primer for amplifying Esherichia coli fru gene
<400> 1
   agctgttgca gccctggcgg taag 24
<210> 2
   <211> 24
   <212> DNA
   <213> Artificial Sequence
<220>
   <223> Description of Artificial Sequence:primer for amplifying Esherichia coli fru gene
<400> 2
   aacaataaaa aagggcagaa aata 24
<210> 3
   <211> 32
   <212> DNA
   <213> Artificial Sequence
<220>
   <223> Description of Artificial Sequence:primer 1 for cloning fruA
<220>
   <221> misc_feature
   <222> (18)
   <223> n=a or g or c or t
<400> 3
   tgcccwaccg gyatygcnca caccttcatg gc 32
<210> 4
   <211> 23
   <212> DNA
   <213> Artificial Sequence
<220>
   <223> Description of Artificial Sequence:primer 2 for cloning fruA
<220>
   <221> misc_feature
   <222> (3,15)
   <223> n=a or g or c or t
<400> 4
   gcngcgaasg gratngcrcc ytc 23
<210> 5
   <211> 16
   <212> DNA
   <213> Artificial Sequence
<220>
   <223> Description of Artificial Sequence:primer M13-20 for sequencing
<400> 5
   gtaaaacgac ggccag 16
<210> 6
   <211> 17
   <212> DNA
   <213> Artificial Sequence
<220>
   <223> Description of Artificial Sequence:primer M13RV for sequencing
<400> 6
   caggaaacag ctatgac 17
<210> 7
   <211> 30
   <212> DNA
   <213> Artificial Sequence
<220>
   <223> Description of Artificial Sequence:primer F3-1 for PCR
<400> 7
   gctaccctgc tgcgcaagaa gctgttcacc 30
<210> 8
   <211> 32
   <212> DNA
   <213> Artificial Sequence
<220>
   <223> Description of Artificial Sequence:primer F3-2 for PCR
<400> 8
   agagcaagaa aacggcaagt cttcctggct gc 32
<210> 9
   <211> 30
   <212> DNA
   <213> Artificial Sequence
<220>
   <223> Description of Artificial Sequence:primer F5-1 for PCR
<400> 9
   tcatcgcggc cttccgcgtt ttgcgtcagg 30
<210> 10
   <211> 30
   <212> DNA
   <213> Artificial Sequence
<220>
   <223> Description of Artificial Sequence:primer F5-2 for PCR
<400> 10
   atccgcagcc atgaaggtgt gagcgatacc 30
<210> 11
   <211> 35
   <212> DNA
   <213> Artificial Sequence
<220>
   <223> Description of Artificial Sequence:primer C1 for PCR
<400> 11
   gtacatattg tcgttagaac gcgtaatacg actca 35
<210> 12
   <211> 35
   <212> DNA
   <213> Artificial Sequence
<220>
   <223> Description of Artificial Sequence:primer C2 PCR
<400> 12
   cgttagaacg cgtaatacga ctcactatag ggaga 35
<210> 13
   <211> 3378
   <212> DNA
   <213> Brevibacterium lactofermentum
<220>
   <221> CDS
   <222> (881)..(2944)
<400> 13
   gtggtaaagg catcaatgtc gcccacgctg tcttgcttgc gggctttgaa accttggctg 60
   tgttcccagc cggcaagctc gaccccttcg tcccactggt ccgcgacatc ggcttgcccg 120
   tggaaactgt tgtgatcaac aacaacgtcc gcaccaacac cacagtcacc gaaccggacg 180
   gcaccaccac caagctcaac ggccccggcg caccgctcag cgagcagaag ctccgtagct 240
   tggaaaaggt gcttatcgac gcgctccgcc ccgaagtcac ctgggttgtc ttggcgggct 300
   cgctgccacc aggggcacca gttgactggt acgcgcgtct caccgcgttg atccattcag 360
   cacgccctga cgttcgcgtg gctgtcgata cctccgacaa gccactgatg gcgttgggcg 420
   agagcttgga tacacctggc gctgctccga acctgattaa gccaaatggt ctggaactgg 480
   gccagctggc taacactgat ggtgaagagc tggaggcgcg tgctgcgcaa ggcgattacg 540
   acgccatcat cgcagctgcg gacgtactgg ttaaccgtgg catcgaacag gtgcttgtca 600
   ccttgggtgc cgctggagcg gtgttggtca acgcagaagg tgcgtggact gctacttctc 660
   caaagattga tgttgtatcc accgttggag ctggagacag tgctcttgca ggttttgtta 720
   tcgcacgttc ccagaagaaa acactggagg aatctctgct gaatgccgtg tcttacggct 780
   cgactgcggc gtctcttcct ggcactacca ttcctcgtcc tgaccaactc gccacaactg 840
   gtgcaacggt cacccaagtc aaaggattga aagaatcagc atg aat agc gta att   895
                                              Met Asn Ser Val Ile
                                               1               5
   aat tcc tcg ctt gtc cgg ctg gat gtc gat ttc ggc gac tcc acc acg   943
   Asn Ser Ser Leu Val Arg Leu Asp Val Asp Phe Gly Asp Ser Thr Thr
                   10                  15                  20
   gat gtc atc aac aac ctt gcc act gtt att ttc gac gct ggc cga gct   991
   Asp Val Ile Asn Asn Leu Ala Thr Val Ile Phe Asp Ala Gly Arg Ala
               25                  30                  35
   tcc tcc gcc gac gcc ctt gcc aaa gac gcg ctg gat cgt gaa gca aag   1039
   Ser Ser Ala Asp Ala Leu Ala Lys Asp Ala Leu Asp Arg Glu Ala Lys
           40                  45                  50
   tcc ggc acc ggt gtc ccc ggt caa gtt gct atc ccc cac tgc cgt tcc   1087
   Ser Gly Thr Gly Val Pro Gly Gln Val Ala Ile Pro His Cys Arg Ser
       55                  60                  65
   gaa gcc gta tct gtc cct acc ttg ggc ttt gct cgc ctg agc aag ggt   1135
   Glu Ala Val Ser Val Pro Thr Leu Gly Phe Ala Arg Leu Ser Lys Gly
   70 75 80 85
   gtg gac ttc agc gga cct gac ggc gat gcc aac ttg gtg ttc ctc att 1183
   Val Asp Phe Ser Gly Pro Asp Gly Asp Ala Asn Leu Val Phe Leu Ile
   90 95 100
   gca gca cct gct ggc ggc ggc aaa gag cac ctg aag atc ctg tcc aaa 1231
   Ala Ala Pro Ala Gly Gly Gly Lys Glu His Leu Lys Ile Leu Ser Lys
   105 110 115
   ctc gct cgc tcc ttg gtg aag aag gat ttc atc aag gct ctg cag gaa 1279
   Leu Ala Arg Ser Leu Val Lys Lys Asp Phe Ile Lys Ala Leu Gln Glu
   120 125 130
   gcc acc acc gag cag gaa atc gtc gac gtt gtc gat gcc gtg ctc aac 1327
   Ala Thr Thr Glu Gln Glu Ile Val Asp Val Val Asp Ala Val Leu Asn
   135 140 145
   cca gca cca aaa acc acc gag cca gct gca gct ccg gct gcg acg gcg 1375
   Pro Ala Pro Lys Thr Thr Glu Pro Ala Ala Ala Pro Ala Ala Thr Ala
   150 155 160 165
   gtt gct gag agt ggg gcg gcg tcg aca agc gtt act cgt atc gtg gca 1423
   Val Ala Glu Ser Gly Ala Ala Ser Thr Ser Val Thr Arg Ile Val Ala
   170 175 180
   atc acc gca tgc cca acc ggt atc gca cac acc tac atg gct gcg gat 1471
   Ile Thr Ala Cys Pro Thr Gly Ile Ala His Thr Tyr Met Ala Ala Asp
   185 190 195
   tcc ctg acg caa aac gcg gaa ggc cgc gat gat gtg gaa ctc gtt gtg 1519
   Ser Leu Thr Gln Asn Ala Glu Gly Arg Asp Asp Val Glu Leu Val Val
   200 205 210
   gag act cag ggc tct tcc gct gtc acc cca gtt gat ccg aag atc atc 1567
   Glu Thr Gln Gly Ser Ser Ala Val Thr Pro Val Asp Pro Lys Ile Ile
   215 220 225
   gaa gct gcc gac gcc gtc atc ttc gcc acc gac gtg gga gtt aaa gac 1615
   Glu Ala Ala Asp Ala Val Ile Phe Ala Thr Asp Val Gly Val Lys Asp
   230 235 240 245
   cgc gag cgt ttc gct ggc aag cca gtc att gaa tcc ggc gtc aag cgc 1663
   Arg Glu Arg Phe Ala Gly Lys Pro Val Ile Glu Ser Gly Val Lys Arg
   250 255 260
   gcg atc aat gag cca gcc aag atg atc gac gag gcc atc gca gcc tcc 1711
   Ala Ile Asn Glu Pro Ala Lys Met Ile Asp Glu Ala Ile Ala Ala Ser
   265 270 275
   aag aac cca aac gcc cgc aag gtt tcc ggt tcc ggt gtc gcg gca tct 1759
   Lys Asn Pro Asn Ala Arg Lys Val Ser Gly Ser Gly Val Ala Ala Ser
   280 285 290
   gct gaa acc acc ggc gag aag ctc ggc tgg ggc aag cgc atc cag cag 1807
   Ala Glu Thr Thr Gly Glu Lys Leu Gly Trp Gly Lys Arg Ile Gln Gln
   295 300 305
   gca gtc atg acc ggc gtg tcc tac atg gtt cca ttc gta gct gcc ggc 1855
   Ala Val Met Thr Gly Val Ser Tyr Met Val Pro Phe Val Ala Ala Gly
   310 315 320 325
   ggc ctc ctg ttg gct ctc ggc ttc gca ttc ggt gga tac gac atg gcg 1903
   Gly Leu Leu Leu Ala Leu Gly Phe Ala Phe Gly Gly Tyr Asp Met Ala
   330 335 340
   aac ggc tgg caa gca atc gcc acc cag ttc tcc ctg acc aac ctg cca 1951
   Asn Gly Trp Gln Ala Ile Ala Thr Gln Phe Ser Leu Thr Asn Leu Pro
   345 350 355
   ggc aac acc gtc gat gtt gac ggc gtg gcc atg acc ttc gag cgt tca 1999
   Gly Asn Thr Val Asp Val Asp Gly Val Ala Met Thr Phe Glu Arg Ser
   360 365 370
   ggc ttc ctg ctg tac ttc ggc gca gtc ctg ttc gct acc ggc caa gca 2047
   Gly Phe Leu Leu Tyr Phe Gly Ala Val Leu Phe Ala Thr Gly Gln Ala
   375 380 385
   gcc atg ggc ttc atc gtg gca gca ctg tct ggc tac acc gca tac gca 2095
   Ala Met Gly Phe Ile Val Ala Ala Leu Ser Gly Tyr Thr Ala Tyr Ala
   390 395 400 405
   ctt gct gga cgc cct ggc atc gcg ccg ggc ttc gtc ggt ggc gcc atc 2143
   Leu Ala Gly Arg Pro Gly Ile Ala Pro Gly Phe Val Gly Gly Ala Ile
   410 415 420
   tcc gtc acc atc ggc gct ggc ttc att ggt ggt ctg gtt acc ggt atc 2191
   Ser Val Thr Ile Gly Ala Gly Phe Ile Gly Gly Leu Val Thr Gly Ile
   425 430 435
   ttg gct ggt ctc att gcc ctg tgg att ggc tcc tgg aag gtg cca cgc 2239
   Leu Ala Gly Leu Ile Ala Leu Trp Ile Gly Ser Trp Lys Val Pro Arg
   440 445 450
   gtg gtg cag tca ctg atg cct gtg gtc atc atc ccg cta ctt acc tca 2287
   Val Val Gln Ser Leu Met Pro Val Val Ile Ile Pro Leu Leu Thr Ser
   455 460 465
   gtg gtt gtt gga ctc gtc atg tac ctc ctg ctg ggt cgc cca ctc gca 2335
   Val Val Val Gly Leu Val Met Tyr Leu Leu Leu Gly Arg Pro Leu Ala
   470 475 480 485
   tcc atc atg act ggt ttg cag gac tgg cta tcg tca atg tcc gga agc 2383
   Ser Ile Met Thr Gly Leu Gln Asp Trp Leu Ser Ser Met Ser Gly Ser
   490 495 500
   tcc gcc atc ttg ctg ggt atc atc ttg ggc ctc atg atg tgt ttc gac 2431
   Ser Ala Ile Leu Leu Gly Ile Ile Leu Gly Leu Met Met Cys Phe Asp
   505 510 515
   ctc ggc gga cca gta aac aag gca gcc tac ctc ttt ggt acc gca ggc 2479
   Leu Gly Gly Pro Val Asn Lys Ala Ala Tyr Leu Phe Gly Thr Ala Gly
   520 525 530
   ctg tct acc ggc gac caa gct tcc atg gaa atc atg gcc gcg atc atg 2527
   Leu Ser Thr Gly Asp Gln Ala Ser Met Glu Ile Met Ala Ala Ile Met
   535 540 545
   gca gct ggc atg gtc cca cca atc gcg ttg tcc att gct acc ctg ctg 2575
   Ala Ala Gly Met Val Pro Pro Ile Ala Leu Ser Ile Ala Thr Leu Leu
   550 555 560 565
   cgc aag aag ctg ttc acc cca gca gag caa gaa aac ggc aag tct tcc 2623
   Arg Lys Lys Leu Phe Thr Pro Ala Glu Gln Glu Asn Gly Lys Ser Ser
   570 575 580
   tgg ctg ctt ggc ctg gca ttc gtc tcc gaa ggt gcc atc cca ttc gcc 2671
   Trp Leu Leu Gly Leu Ala Phe Val Ser Glu Gly Ala Ile Pro Phe Ala
   585 590 595
   gca gct gac cca ttc cgt gtg atc cca gca atg atg gct ggc ggt gca 2719
   Ala Ala Asp Pro Phe Arg Val Ile Pro Ala Met Met Ala Gly Gly Ala
   600 605 610
   acc act ggt gca att tcc atg gca ctg ggc gtc ggc tct cgg gct cca 2767
   Thr Thr Gly Ala Ile Ser Met Ala Leu Gly Val Gly Ser Arg Ala Pro
   615 620 625
   cac ggc ggt atc ttc gtg gtc tgg gca atc gaa cca tgg tgg ggc tgg 2815
   His Gly Gly Ile Phe Val Val Trp Ala Ile Glu Pro Trp Trp Gly Trp
   630 635 640 645
   ctc atc gca ctt gca gca ggc acc atc gtg tcc acc atc gtt gtc atc 2863
   Leu Ile Ala Leu Ala Ala Gly Thr Ile Val Ser Thr Ile Val Val Ile
   650 655 660
   gca ctg aag cag ttc tgg cca aac aag gcc gtc gct gca gaa gtc gcg 2911
   Ala Leu Lys Gln Phe Trp Pro Asn Lys Ala Val Ala Ala Glu Val Ala
   665 670 675
   aag caa gaa gca gct gcg gcc gcc gta gcc gca taaccctgat gtctggtcgg 2964
   Lys Gln Glu Ala Ala Ala Ala Ala Val Ala Ala
   680 685
   acattgtttt tgcttccggt aacgtggcaa aacgaacaat gtctcactag actaaagtga 3024
   gatccacatt aaatcccctc cgttgggggt ttaactaaca aatcgctgcg ccctaatccg 3084
   ttcggatgaa cggcgtagca acacgaaagg acactttcca tggcttccaa gactgtaacc 3144
   gtcggttcct ccgttggcct gcacgcacgt ccagcatcca tcatcgctga agcggctgct 3204
   gagtacgacg acgaaatctt gctgaccctg gttggctccg atgatgacga agagaccgac 3264
   gcttcctctt ccctcatgat catggcgctg ggtgcagagc acggcaacga agtaaccgtc 3324
   acctccgaca acgctgaagc tgttgagaag atcgctgcgc ttatcgcaca ggac 3378
<210> 14
   <211> 688
   <212> PRT
   <221> CDS
<400> 14
   Met Asn Ser Val Ile Asn Ser Ser Leu Val Arg Leu Asp Val Asp Phe
   1 5 10 15
   Gly Asp Ser Thr Thr Asp Val Ile Asn Asn Leu Ala Thr Val Ile Phe
   20 25 30
   Asp Ala Gly Arg Ala Ser Ser Ala Asp Ala Leu Ala Lys Asp Ala Leu
   35 40 45
   Asp Arg Glu Ala Lys Ser Gly Thr Gly Val Pro Gly Gln Val Ala Ile
   50 55 60
   Pro His Cys Arg Ser Glu Ala Val Ser Val Pro Thr Leu Gly Phe Ala
   65 70 75 80
   Arg Leu Ser Lys Gly Val Asp Phe Ser Gly Pro Asp Gly Asp Ala Asn
   85 90 95
   Leu Val Phe Leu Ile Ala Ala Pro Ala Gly Gly Gly Lys Glu His Leu
   100 105 110
   Lys Ile Leu Ser Lys Leu Ala Arg Ser Leu Val Lys Lys Asp Phe Ile
   115 120 125
   Lys Ala Leu Gln Glu Ala Thr Thr Glu Gln Glu Ile Val Asp Val Val
   130 135 140
   Asp Ala Val Leu Asn Pro Ala Pro Lys Thr Thr Glu Pro Ala Ala Ala
   145 150 155 160
   Pro Ala Ala Thr Ala Val Ala Glu Ser Gly Ala Ala Ser Thr Ser Val
   165 170 175
   Thr Arg Ile Val Ala Ile Thr Ala Cys Pro Thr Gly Ile Ala His Thr
   180 185 190
   Tyr Met Ala Ala Asp Ser Leu Thr Gln Asn Ala Glu Gly Arg Asp Asp
   195 200 205
   Val Glu Leu Val Val Glu Thr Gln Gly Ser Ser Ala Val Thr Pro Val
   210 215 220
   Asp Pro Lys Ile Ile Glu Ala Ala Asp Ala Val Ile Phe Ala Thr Asp
   225 230 235 240
   Val Gly Val Lys Asp Arg Glu Arg Phe Ala Gly Lys Pro Val Ile Glu
   245 250 255
   Ser Gly Val Lys Arg Ala Ile Asn Glu Pro Ala Lys Met Ile Asp Glu
   260 265 270
   Ala Ile Ala Ala Ser Lys Asn Pro Asn Ala Arg Lys Val Ser Gly Ser
   275 280 285
   Gly Val Ala Ala Ser Ala Glu Thr Thr Gly Glu Lys Leu Gly Trp Gly
   290 295 300
   Lys Arg Ile Gln Gln Ala Val Met Thr Gly Val Ser Tyr Met Val Pro
   305 310 315 320
   Phe Val Ala Ala Gly Gly Leu Leu Leu Ala Leu Gly Phe Ala Phe Gly
   325 330 335
   Gly Tyr Asp Met Ala Asn Gly Trp Gln Ala Ile Ala Thr Gln Phe Ser
   340 345 350
   Leu Thr Asn Leu Pro Gly Asn Thr Val Asp Val Asp Gly Val Ala Met
   355 360 365
   Thr Phe Glu Arg Ser Gly Phe Leu Leu Tyr Phe Gly Ala Val Leu Phe
   370 375 380
   Ala Thr Gly Gln Ala Ala Met Gly Phe Ile Val Ala Ala Leu Ser Gly
   385 390 395 400
   Tyr Thr Ala Tyr Ala Leu Ala Gly Arg Pro Gly Ile Ala Pro Gly Phe
   405 410 415
   Val Gly Gly Ala Ile Ser Val Thr Ile Gly Ala Gly Phe Ile Gly Gly
   420 425 430
   Leu Val Thr Gly Ile Leu Ala Gly Leu Ile Ala Leu Trp Ile Gly Ser
   435 440 445
   Trp Lys Val Pro Arg Val Val Gln Ser Leu Met Pro Val Val Ile Ile
   450 455 460
   Pro Leu Leu Thr Ser Val Val Val Gly Leu Val Met Tyr Leu Leu Leu
   465 470 475 480
   Gly Arg Pro Leu Ala Ser Ile Met Thr Gly Leu Gln Asp Trp Leu Ser
   485 490 495
   Ser Met Ser Gly Ser Ser Ala Ile Leu Leu Gly Ile Ile Leu Gly Leu
   500 505 510
   Met Met Cys Phe Asp Leu Gly Gly Pro Val Asn Lys Ala Ala Tyr Leu
   515 520 525
   Phe Gly Thr Ala Gly Leu Ser Thr Gly Asp Gln Ala Ser Met Glu Ile
   530 535 540
   Met Ala Ala Ile Met Ala Ala Gly Met Val Pro Pro Ile Ala Leu Ser
   545 550 555 560
   Ile Ala Thr Leu Leu Arg Lys Lys Leu Phe Thr Pro Ala Glu Gln Glu
   565 570 575
   Asn Gly Lys Ser Ser Trp Leu Leu Gly Leu Ala Phe Val Ser Glu Gly
   580 585 590
   Ala Ile Pro Phe Ala Ala Ala Asp Pro Phe Arg Val Ile Pro Ala Met
   595 600 605
   Met Ala Gly Gly Ala Thr Thr Gly Ala Ile Ser Met Ala Leu Gly Val
   610 615 620
   Gly Ser Arg Ala Pro His Gly Gly Ile Phe Val Val Trp Ala Ile Glu
   625 630 635 640
   Pro Trp Trp Gly Trp Leu Ile Ala Leu Ala Ala Gly Thr Ile Val Ser
   645 650 655
   Thr Ile Val Val Ile Ala Leu Lys Gln Phe Trp Pro Asn Lys Ala Val
   660 665 670
   Ala Ala Glu Val Ala Lys Gln Glu Ala Ala Ala Ala Ala Val Ala Ala
   675 680 685

## Description

### Technical Field

The present invention relates to methods for producing L-amino acids by fermentation, in particular, methods for producing L-lysine and L-glutamic acid. There are widely used L-lysine as additive for animal feed and so forth, and L-glutamic acid as raw materials of seasonings and so forth.

### Background Art

L-Amino acids such as L-lysine and L-glutamic acid are industrially produced by fermentation by using coryneform bacteria that belong to the genus *Brevibacterium, Coryrnebacterium* or the like and have abilities to produce these L-amino acids. In order to improve the productivity of these coryneform bacteria, strains isolated from nature or artificial mutants of such strains have been used.

Further, various techniques have been disclosed for increasing the L-amino acid producing abilities by using recombinant DNA techniques to enhance L-amino acid biosynthetic enzymes. For example, as for coryneform bacteria having L-lysine producing ability, it is known that the L-lysine producing ability of the bacteria can be improved by introduction of a gene coding for aspartokinase of which feedback inhibition by L-lysine and L-threonine is desensitised (mutant type *lysC*), dihydrodipicolinate reductase gene (*dapB*), dihydrodipicolinate synthase gene (*dapA*), diaminopimelate decarboxylase gene (*lysA*) and diaminopimelate dehydrogenase gene (*ddh*) (WO96/40934), *lysA* and *ddh* (Japanese Patent Laid-open Publication No. (Kokai) No. 9-322774), *lysC, lysA* and phosphoenolpyruvate carboxylase gene (*ppc*) (Japanese Patent Laid-open Publication No. No. 10-165180), mutant type *lysC, dapB, dapA, lysA* and aspartate aminotransferase gene (*aspC*) (Japanese Patent Laid-open Publication No. 10-215883).

Further, as for *Escherichia* bacteria, it is known that the L-lysine producing ability is improved by successively enhancing *dapA*, mutant type *ysC. dapB* and diaminopimelate dehydrogenase gene (*ddh*) (or tetrahydrodipicolinate succinylase gene (*dapD*) and succinyl diaminopimelate deacylase gene (*dapE*)) (WO 95/16042). Incidentally, in WO95/16042, tetrahydrodipicolinate succinylase is erroneously described as succinyl diaminopimelate transaminase.

Furthermore, it was reported that introduction of a gene coding for citrate synthase derived from *Escherichia coli* or *Corynebacterium glutamicum* was effective for enhancement of L-glutamic acid producing ability in *Corynebacterium* or *Brevibacterium* bacteria (Japanese Patent Publication (Kokoku) No. 7-121228). In addition, Japanese Patent Laid-open Publication No. 61-268185 discloses a cell harboring recombinant DNA containing a glutamate dehydrogenase gene derived from *Corynebacterium* bacteria. Furthermore, Japanese Patent Laid-open Publication No. 63-214189 discloses a technique for increasing L-glutamic acid producing ability by amplifying glutamate dehydrogenase gene, isocitrate dehydrogenase gene, aconitate hydratase gene and citrate synthase gene.

However, structure of a gene coding for fructose phosphotransferase has not been reported for coryneform bacteria, and utilization of a gene coding for fructose phosphotransferase for breeding of coryneform bacteria is also unknown so far.

In addition, a gene coding for fructose phosphotransferase of coryneform bacteria such as *Brevibacterium* bacteria has not been known.

### Disclosure of the Invention

An object of the present invention is to provide a method for producing an L-amino acid such as L-lysine or L-glutamic acid by fermentation, which is further improved compared with conventional techniques,

The inventors of the present invention assiduously studies in order to achieve the aforementioned object. As a result, they found that, if a gene coding for fructose phosphotransferase was introduced into a coryneform bacterium to amplify the fructose phosphotransferase activity, production amount of L-lysine or L-glutamic acid could be increased. Further, they also succeeded in isolating a gene coding for fructose phosphotransferase of *Brevibacterium lactofermentum*. Thus, they accomplished the present invention.

That is, the present invention provides the followings.
(1) A method for producing an L-amino acid, comprising the steps of culturing a coryneform bacterium having enhanced intracellular fructose phosphotransferase activity and an ability to produce an L-amino acid in a medium to produce and accumulate the L-amino acid in the culture and collecting the L-amino acid from the culture wherein the fructose phosphotransferase activity is enhanced by increasing copy number of a gene coding for fructose phosphotransferase in a cell of the bacterium or by replacing an expression regulatory sequence of the gene.
(2) The method according to (1), wherein the gene coding for fructose phosphotransferase is derived from an *Escherichia* bacterium.
(3) The method according to (1), wherein the gene coding for fructose phosphotransferase is derived from a coryneform bacterium.
(4) The method according to any one of (1) to (3), wherein the L-amino acid is selected from L-lysine, L-glutamic acid, L-threonine, L-isoleucine and L-serine.
(5) The method according to (4), wherein the L-amino acid is selected from L-lysine and L-glutamic acid.
(6) The method according to any one of (1) to (5), wherein the medium contains fructose as a carbon source.

Hereafter, the present invention will be explained in detail.

### <1> Coryneform bacterium used in the method of the present invention

The coryneform bacterium used in the method of the present invention is a coryneform bacterium having an L-amino acid producing ability and enhanced intracellular fructose phosphotransferase activity. The L-amino acid may be L-lysine, L-glutamic acid, L-threonine, L-isoleucine, L-serine or the like. Among these, L-lysine and L-glutamic acid are preferred. Although embodiments of the present invention will be explained hereafter mainly for coryneform bacteria having L-lysine producing ability or L-glutamic acid producing ability, the present invention can be similarly used for any L-amino acid so long as the proper biosynthesis system of the desired L-amino acid locates downstream from fructose phosphotransferase.

The coryneform bacteria referred to in the present invention include the group of microorganisms defined in Bergey's Manual of Determinative Bacteriology, 8th edition, p.599 (1974), which are aerobic, gram-positive and non-acid-fast bacilli not showing sporogenesis ability. They include those having hitherto been classified into the genus *Brevibacterium*, but united into the genus *Corynebacterium* at present (Int. J. Syst. Bacteriol., 41, 255 (1981)), and also include bacteria belonging to the genus *Brevibacterium* or *Microbacterium* closely relative to the genus *Corynebacterium*. Examples of coryneform bacterium strain suitably used for the production of L-lysine or L-glutamic acid include, for example, the followings.
*Corynebacterium acetoacidophilum* ATCC 13870
*Corynebacterium acetoglutamicum* ATCC 15806
*Corynebacterium callunae* ATCC 15991
*Corynebacterium glutamicum* ATCC 13032
(*Brevibacterium divaricatum*) ATCC 14020
(*Brevibacterium lactofermentum)* ATCC 13869
*(Corynebacterium lilium)* ATCC 15990
*(Brevibacterium flavum)* ATCC 14067
*Corynebacterium melassecola* ATCC 17965
*Brevibacterium saccharolyticum* ATCC 14066
*Brevibacterium immariophilum* ATCC 14068
*Brevibacterium roseum* ATCC 13825
*Brevibacterium thiogenitalis* ATCC 19240
*Microbacterium ammoniaphilum* ATCC 15354
*Corynebacterium thermoaminogenes* AJ12340 (FERM BP-1539)

To obtain these strains, one can be provided them from, for example, the American Type Culture Collection (Address: 12301 Parklawn Drive, Rockville, Maryland 20852, United States of America). That is, each strain is assigned its registration number, and one can request provision of each strain by utilizing its registration number. The registration numbers corresponding to the strains are indicated on the catalog of the American Type Culture Collection. Further, the AJ12340 strain was deposited at the National Institute of Bioscience and Human-Technology, Agency of Industrial Science and Technology, Ministry of International Trade and Industry (1-3 Higashi 1-Chome, Tsukuba-shi, Ibaraki-ken, Japan, postal code: 305-8566)) as an international deposit under the provisions of the Budapest Treaty.

Besides the aforementioned strains, mutant strains derived from these bacterial strains and having an ability to produce an L-amino acid such as L-lysine or L-glutamic acid can also be used for the present invention. Examples of such artificial mutant strains include mutant strains resistant to S-(2-aminoethyl)-cysteine (abbreviated as "AEC" hereinafter) (e.g., *Brevibacterium lactofermentum* AJ11082 (NRRL B-11470), refer to Japanese Patent Publication (Kokoku) Nos. 56-1914, 56-1915, 57-14157, 57-14158, 57-30474, 58-10075, 59-4993, 61-35840, 62-24074, 62-36673, 5-11958, 7-112437 and 7-112438), mutant strains requiring amino acids such as L-homoserine for their growth (Japanese Patent Publication Nos. 48-28078 and 56-6499), mutant strains resistant to AEC and further requiring amino acids such as L-leucine, L-homoserine, L-proline, L-serine, L-arginine, L-alanine and L-valine (U.S. Patent Nos. 3,708,395 and 3,825,472), L-lysine producing mutant strains resistant to DL-α-amino-ε-caprolactam, α-amino-lauryllactam, aspartic acid analogue, sulfa drug, quinoid and N-lauroylleucine, L-lysine producing mutant strains resistant to oxaloacetate decarboxylase or a respiratory tract enzyme inhibitor (Japanese Patent Laid-open Publication Nos. 50-53588, 50-31093, 52-102498, 53-9394, 53-86089, 55-9783, 55-9759, 56-32995, 56-39778, Japanese Patent Publication Nos. 53-43591 and 53-1833), L-lysine producing mutant strains requiring inositol or acetatic acid (Japanese Patent Laid-open Publication Nos. 55-9784 and 56-8692), L-lysine producing mutant strains that are susceptible to fluoropyruvic acid or a temperature of 34°C or higher (Japanese Patent Laid-open Publication Nos. 55-9783 and 53-86090), L-lysine producing mutant strains of *Brevibacterium* or *Corynebacterium* bacteria resistant to ethylene glycol (U.S. Patent No. 4,411,997) and so forth.

Further, there can also be mentioned *Corynebacterium acetoacidophilum* AJ12318 (FERM BP-1172) (refer to U.S. Patent No. 5,188,949) and so forth as coryneform bacteria having L-threonine producing ability, and *Brevibacterium flavum* AJ12149 (FERM BP-759) (refer to U.S. Patent No. 4,656,135) and so forth as coryneform bacteria having L-isoleucine producing ability.

### <2> Amplification of fructose phosphotransferase activity

In order to amplify fructose phosphotransferase activity in a cell of coryneform bacterium, a recombinant DNA can be prepared by ligating a gene fragment coding for fructose phosphotransferase with a vector functioning in the bacterium, preferably a multicopy vector, and introduced into a coryneform bacterium having an ability to produce L-lysine or L-glutamic acid to transform it. The copy number of the gene coding for fructose phosphotransferase in the cell of the transformant strain is thereby increased, and as a result, the fructose phosphotransferase activity is amplified. In *Escherichia coli*, fructose phosphotransferase is encoded by *fruA* gene.

Although the fructose phosphotransferase gene is preferably a gene derived from a coryneform bacterium, any of such genes derived from other organisms such as *Escherichia* bacteria can also be used.

The nucleotide sequence of *fruA* gene of *Escherichia coli* was already elucidated (Genbank/EMBL/DDBJ accession No. M23196), and therefore the *fruA* gene can be obtained by PCR (polymerase chain reaction, refer to White, T.J. et al., Trends Genet.5, 185 (1989)) using primers prepared based on the nucleotide sequence, for example, the primers shown in Sequence Listing as SEQ ID NOS: 1 and 2 and chromosomal DNA of *Escherichia coli* as a template.

Further, the *fruA* gene derived from a coryneform bacterium such as *Brevibacterium lactofermentum* can also be obtained as a partial sequence by selecting a region showing high homology among amino acid sequences expected from known *fruA* genes such as those of *Bacillus subtilis, Escherichia coli, Mycoplasma genitalium* and *Xanthomonas compestris*, preparing primers for PCR based on the amino acid sequence of that region and performing PCR using *Brevibacterium lactofermentum* as a template. As examples of the aforementioned primers, the oligonucleotides shown as SEQ ID NO: 3 and SEQ ID NO: 4 can be mentioned.

Then, by utilizing the partial sequence of the *fruA* gene obtained as described above, the 5' unknown region and 3' unknown region of the *fruA* gene are obtained by means of inverse PCR (Genetics, 120, 621-623 (1988)), a method using LA-PCR In Vitro Cloning Kit (Takara Shuzo) or the like. When LA-PCR In Vitro Cloning Kit is used, the 3' unknown region of *fruA* gene can be obtained by, for example, performing PCR using the primers shown as SEQ ID NOS: 5 and 9 as primary PCR and PCR using the primers shown as SEQ ID NOS: 6 and 10 as secondary PCR. Further, the 5' unknown region of *fruA* gene can be obtained by, for example, performing PCR using the primers shown as SEQ ID NOS: 7 and 9 as primary PCR and PCR using the primers shown as SEQ ID NOS: 8 and 10 as secondary PCR. The nucleotide sequence of the DNA fragment including the full length of *fruA* gene obtained as described above is shown as SEQ ID NO: 13. Further, the amino acid sequence translated from an open reading frame deduced from the above nucleotide sequence is shown as SEQ ID NO: 14.

Furthermore, since the *fruA* gene of *Brevibacterium lactofermentum* and the nucleotide sequences of the flanking regions thereof are elucidated by the present invention, a DNA fragment containing the full length of the *fruA* gene can be obtained by PCR using oligonucleotides designed based on the nucleotide sequences of those flanking regions.

Genes coding for fructose phosphotransferase of other bacteria can also be obtained in a similar manner.

The *fruA* used in the method gene of the present invention may be one coding for fructose phosphotransferase including substitution, deletion, insertion, addition or inversion of one or several amino acids at one or more sites, so long as the fructose phosphotransferase activity of the encoded protein is not degraded. Although the number of "several" amino acids referred to herein differs depending on position or type of amino acid residues in the three-dimensional structure of the protein, it may be specifically 2 to 200, preferably 2 to 50, more preferably 2 to 20.

A DNA coding for the substantially same protein as the aforementioned fructose phosphotransferase can be obtained by, for example, modifying the nucleotide sequence of *fruA* by means of the site-directed mutagenesis method so that one or more amino acid residues at a specified site should involve substitution, deletion, insertion, addition or inversion. A DNA modified as described above may also be obtained by a conventionally known mutagenesis treatment. The mutagenesis treatment includes a method of treating a DNA before the mutagenesis treatment in vitro with hydroxylamine or the like, and a method for treating a microorganism such as an *Escherichia* bacterium harboring a DNA before the mutagenesis treatment by ultraviolet irradiation or with a mutagenizing agent used for a usual mutagenesis treatment such as N-methyl-N'-nitro-N-nitrosoguanidine (NTG) and nitrous acid.

A DNA coding for substantially the same protein as fructose phosphotransferase can be confirmed by expressing such a DNA having a mutation as described above in an appropriate cell, and investigating activity of the expressed product. A DNA coding for substantially the same protein as fructose phosphotransferase can also be obtained by isolating a DNA that is hybridizable with a probe having a nucleotide sequence comprising, for example, the nucleotide sequence corresponding to nucleotide numbers of 881 to 2944 of the nucleotide sequence shown in Sequence Listing as SEQ ID NO: 13 or a part thereof, under the stringent conditions, and codes for a protein having the fructose phosphotransferase activity from a DNA coding for fructose phosphotransferase having a mutation or from a cell harboring it. The "stringent conditions" referred to herein are conditions under which so-called specific hybrid is formed, and nonspecific hybrid is not formed. It is difficult to clearly express these conditions by using any numerical value. However, for example, the stringent conditions are exemplified by a condition under which DNAs having high homology, for example, DNAs having homology of not less than 50% are hybridized with each other, but DNAs having homology lower than the above are not hybridized with each other. Alternatively, the stringent conditions are exemplified by a condition under which DNAs are hybridized with each other at a salt concentration corresponding to an ordinary condition of washing in Southern hybridization, i.e., 1 x SSC, 0.1% SDS, preferably 0.1 x SSC, 0.1% SDS, at 60°C.

As the probe, a partial sequence of the nucleotide sequence of SEQ ID NO: 13 can also be used. Such a probe may be prepared by PCR using oligonucleotides produced based on the nucleotide sequence of SEQ ID NO: 13 as primers, and a DNA fragment containing the nucleotide sequence of SEQ ID NO: 13 as a template. When a DNA fragment in a length of about 300 bp is used as the probe, the conditions of washing for the hybridization consist of, for example, 50°C, 2 x SSC and 0.1% SDS.

Genes that are hybridizable under such conditions as described above includes those having a stop codon in the genes, and those having no activity due to mutation of active center. However, such genes can be easily distinguished by ligating each gene with a commercially available activity expression vector, and measuring the fructose phosphotransferase activity by the method described in Mori, M. & Shiio, I., Agric. Biol. Chem., 51, 129-138 (1987).

Specific examples of the DNA coding for a protein substantially the same as fructose phosphotransferase include a DNA coding for a protein that has homology of preferably 55% or more, more preferably 60% or more, still more preferably 80% or more, with respect to the amino acid sequence shown as SEQ ID NO: 14 and has fructose phosphotransferase activity.

The chromosomal DNA can be prepared from a bacterium, which is a DNA donor, for example, by the method of Saito and Miura (refer to H. Saito and K. Miura, Biochem. Biophys. Acta, 72, 619 (1963); Text for Bioengineering Experiments, Edited by the Society for Bioscience and Bioengineering, Japan, pp.97-98, Baifukan, 1992) or the like.

If the gene coding for fructose phosphotransferase amplified by the PCR method is ligated to a vector DNA autonomously replicable in a cell of *Escherichia coli* and/or coryneform bacteria to prepare a recombinant DNA and this is introduced into *Escherichia coli*, subsequent procedures become easy. As the vector autonomously replicable in a cell of *Escherichia coli,* a plasmid vector, especially such a vector autonomously replicable in a cell of host is preferred, and examples of such a vector include pUC19, pUC18, pBR322, pHSG299, pHSG399, pHSG398, RSF1010 and so forth.

Examples of the vector autonomously replicable in a cell of coryneform bacteria include pAM330 (refer to Japanese Patent Laid-open Publication No. 58-67699), pHM1519 (refer to Japanese Patent Laid-open Publication No. 58-77895) and so forth. Moreover, if a DNA fragment having an ability to make a plasmid autonomously replicable in coryneform bacteria is taken out from these vectors and inserted into the aforementioned vectors for *Escherichia coli*, they can be used as a so-called shuttle vector autonomously replicable in both of *Escherichia coli* and coryneform bacteria. Examples of such a shuttle vector include those mentioned below. There are also indicated microorganisms that harbor each vector, and accession numbers thereof at the international depositories are shown in the parentheses, respectively.
pAJ655 *Escherichia coli* AJ11882 (FERM BP-136)
   *Corynehacterium glutamicum* SR8201 (ATCC 39135)
pAJ1844 *Escherichia coli* AJ11883 (FERM BP-137)
   *Corynebacterium glutamicum* SR8202 (ATCC 39136)
pAJ611 *Escherichia coli* AJ11884 (FERM BP-138)
pAJ3148 *Corynebacterium glutamicum* SR8203 (ATCC 39137)
pAJ440 *Bacillus subtilis* AJ11901 (FERM BP-140)
pHC4 *Escherichia coli* AJ12617 (FERM BP-3532)

In order to prepare a recombinant DNA by ligating a gene coding for fructose phosphotransferase and a vector that can function in a cell of coryneform bacterium, the vector is digested with a restriction enzyme corresponding to the terminus of the gene coding for fructose phosphotransferase. Ligation is usually performed by using a ligase such as T4 DNA ligase.

To introduce the recombinant DNA prepared as described above into a microorganism, any known transformation methods that have hitherto been reported can be employed. For instance, employable are a method of treating recipient cells with calcium chloride so as to increase the permeability of DNA, which has been reported for *Escherichia coli* K-12 (Mandel, M. and Higa, A., J. Mol. Biol., 53, 159 (1970)), and a method of preparing competent cells from cells which are at the growth phase followed by introducing the DNA thereinto, which has been reported for *Bacillus subtilis* (Duncan, C.H., Wilson, G.A. and Young, F.E., Gene, 1, 153 (1977)). In addition to these, also employable is a method of making DNA-recipient cells into protoplasts or spheroplasts, which can easily take up recombinant DNA, followed by introducing the recombinant DNA into the cells, which is known to be applicable to *Bacillus subtilis*, actinomycetes and yeasts (Chang, S. and Choen, S.N., Molec. Gen. Genet., 168, 111 (1979); Bibb, M.J., Ward, J.M. and Hopwood, O.A., Nature, 274, 398 (1978); Hinnen, A., Hicks, J.B. and Fink, G.R., Proc. Natl. Sci., USA, 75, 1929 (1978)). The transformation method used in the examples mentioned in the present specification is the electric pulse method (refer to Japanese Patent Laid-open No. 2-207791).

Amplification of the fructose phosphotransferase activity can also be achieved by introducing multiple copies of a gene coding for fructose phosphotransferase into chromosomal DNA of the host. In order to introduce multiple copies of the gene coding for fructose phosphotransferase into chromosomal DNA of a microorganism belonging to coryneform bacteria, homologous recombination is carried out by using a sequence whose multiple copies exist in the chromosomal DNA as targets. As sequences whose multiple copies exist in the chromosomal DNA, repetitive DNA or inverted repeats existing at the end of a transposable element can be used. Further, as disclosed in Japanese Patent Laid-open Publication No. 2-109985, it is also possible to incorporate the gene coding for fructose phosphotransferase into transposon, and allow it to be transferred to introduce multiple copies of the gene into the chromosomal DNA. According to any of these methods, the fructose phosphotransferase is amplified as a result of increase of copy number of the gene cording for fructose phosphotransferase in the transformant strain.

The amplification of fructose phosphotransferase activity can also be attained by, besides being based on the aforementioned gene amplification, replacing an expression regulatory sequence such as a promoter of the gene coding for fructose phosphotransferase on chromosomal DNA or plasmid with a stronger one (see Japanese Patent Laid-open Publication No. 1-215280). For example, lac promoter, trp promoter, trc promoter, tac promoter, P_{R} promoter and P_{L} promoter of lambda phage and so forth are known as strong promoters. Substitution of these promoters enhances expression of the gene coding for fructose phosphotransferase, and hence the fructose phosphotransferase activity is amplified.

In the coryneform bacterium used in the method of the present invention, in addition to the enhancement of fructose phosphotransferase activity, another enzyme involved in a biosynthetic pathway of another amino acid or the glycolysis system may also be enhanced by enhancing a gene for the enzyme. For example, examples of genes that can be used for production of L-lysine include a gene coding for the aspartokinase α-subunit protein or β-subunit protein of which synergistic feedback inhibition by L-lysine and L-threonine is desensitised (International Patent Publication WO94/25605), wild type phosphoenolpyruvate carboxylase gene derived from coryneform bacterium (Japanese Patent Laid-open Publication No. 60-87788), gene coding for wild type dihydrodipicolinate synthetase derived from coryneform bacterium (Japanese Patent Publication No. 6-55149) and so forth.

Further, examples of genes that can be used for production of L-glutamic acid include genes of glutamate dehydrogenase (GDH, Japanese Patent Laid-open Publication No. 61-268185), glutamine synthetase, glutamate synthase, isocitrate dehydrogenase (Japanese Patent Laid-open Publication Nos. 62-166890 and 63-214189), aconitate hydratase (Japanese Patent Laid-open Publication No. 62-294086), citrate synthase, pyruvate carboxylase (Japanese Patent Laid-open Publication Nos. 60-87788 and 62-55089), phosphoenolpyruvate carboxylase, phosphoenolpyruvate synthase, fructose phosphotransferase, phosphoglyceromutase, phosphoglycerate kinase, glyceraldehyde-3-phosphate dehydrogenase, triose phosphate isomerase, fructose bisphosphate aldolase, phosphofructokinase (Japanese Patent Laid-open Publication No. 63-102692), glucosephosphate isomerase and so forth.

Further, activity of an enzyme that catalyzes a reaction for producing a compound other than the desired L-amino acid by branching off from the biosynthetic pathway of the L-amino acid may be decreased or made deficient. For example, examples of an enzyme that catalyzes a reaction for producing a compound other than L-lysine by branching off from the biosynthetic pathway of L-lysine include homoserine dehydrogenase (refer to WO95/23864). Further, examples of an enzyme that catalyzes a reaction for producing a compound other than L-glutamic acid by branching off from the biosynthetic pathway of L-glutamic acid include α-ketoglutarate dehydrogenase, isocitrate lyase, phosphate acetyltransferase, acetate kinase, acetohydroxy acid synthase, acetolactate synthase, formate acetyltransferase, lactate dehydrogenase, glutamate decarboxylase, 1-pyrrolin dehydrogenase and so forth.

Furthermore, by imparting a temperature sensitive mutation for a biotin action suppressing substance such as surfactants to a coryneform bacterium having L-glutamic acid producing ability, L-glutamic acid can be produced in a medium containing an excessive amount of biotin in the absence of a biotin action suppressing substance (refer to WO96/06180). As an example of such a coryneform bacterium, the *Brevibacterium lactofermentum* AJ13029 strain disclosed in WO96/06180 can be mentioned. The AJ13029 strain was deposited at the Institute of Bioscience and Human-Technology, Agency of Industrial Science and Technology (1-3 Higashi 1-Chome, Tsukuba-shi, Ibaraki-ken, Japan, postal code: 305-8566) on September 2, 1994, and given with an accession number of PERM P-14501, and then it was transferred to an international deposit under the provisions of the Budapest Treaty on August 1, 1995, and given with an accession number of FERM BP-5189.

Furthermore, by imparting a temperature sensitive mutation for a biotin action suppressing substance such as surfactants to a coryneform bacterium having L-lysine and L-glutamic acid producing abilities, L-lysine and L-glutamic acid can be simultaneously produced in a medium containing an excessive amount of biotin in the absence of a biotin action suppressing substance (refer to WO96/06180). As an example of such a coryneform bacterium, the *Brevibacterium lactofermentum* AJ12933 strain disclosed in WO96/06180 can be mentioned. The AJ12933 strain was deposited at the Institute of Bioscience and Human-Technology, Agency of Industrial Science and Technology (1-3 Higashi 1-Chome, Tsukuba-shi, Ibaraki-ken, Japan, postal code: 305-8566) on June 3, 1994, and given with an accession number of FERM P-14348, then it was transferred to an international deposit under the provisions of the Budapest Treaty on August 1, 1995, and given with an accession number of FERM BP-5188.

### <3> Production of L-amino acid

If a coryneform bacterium having amplified fructose phosphotransferase activity and an L-amino acid producing ability is cultured in a suitable medium, the L-amino acid is accumulated in the medium. For example, if a coryneform bacterium having amplified fructose phosphotransferase activity and L-lysine producing ability is cultured in a suitable medium, L-lysine is accumulated in the medium. Further, if a coryneform bacterium having amplified fructose phosphotransferase activity and L-glutamic acid producing ability is cultured in a suitable medium, L-glutamic acid is accumulated in the medium.

Furthermore, if a coryneform bacterium having amplified fructose phosphotransferase activity and L-lysine and L-glutamic acid producing abilities is cultured in a suitable medium, L-lysine and L-glutamic acid are accumulated in the medium. When L-lysine and L-glutamic acid are simultaneously produced by fermentation, an L-lysine producing bacterium may be cultured under an L-glutamic acid producing condition, or a coryneform bacterium having L-lysine producing ability and a coryneform bacterium having L-glutamic acid producing ability can be cultured as mixed culture (Japanese Patent Laid-open Publication No. No. 5-3793).

The medium used for producing L-amino acids such as L-lysine and L-glutamic acid by using the microorganism used in the method of the present invention is a usual medium that contains a carbon source, a nitrogen source, inorganic ions and other organic trace nutrients as required. As the carbon source, it is possible to use hydrocarbons such as glucose, lactose, galactose, fructose, sucrose, blackstrap molasses and starch hydrolysate; alcohols such as ethanol and inositol; or organic acids such as acetic acid, fumaric acid, citric acid and succinic acid. In the present invention, fructose is particularly preferred among these. Usually, in the production of L-amino acids by fermentation using coryneform bacteria, yield tends to be degraded if fructose is used as a carbon source of the medium. However, the microorganism used in the method of the present invention efficiently produces an L-amino acid in a medium containing fructose as a carbon source. This effect is particularly remarkable in L-lysine production.

As the nitrogen source, there can be used inorganic or organic ammonium salts such as ammonium sulfate, ammonium nitrate, ammonium chloride, ammonium phosphate and ammonium acetate, ammonia, organic nitrogen such as peptone, meat extract, yeast extract, corn steep liquor and soybean hydrolysate, ammonia gas, aqueous ammonia and so forth.

As the inorganic ions (or sources thereof), added is a small amount of potassium phosphate, magnesium sulfate, iron ions, manganese ions and so forth. As for the organic trace nutrients, it is desirable to add required substances such as vitamin B₁, yeast extract and so forth in a suitable amount as required.

The culture is preferably performed under an aerobic condition attained by shaking, stirring for aeration or the like for 16 to 72 hours. The culture temperature is controlled to be at 30°C to 45°C, and pH is controlled to be 5 to 9 during the culture. For such adjustment of pH, inorganic or organic acidic or alkaline substances, ammonia gas and so forth can be used.

Collection of L-amino acid from fermentation broth can be attained in the same manner as in usual production methods of L-amino acids. For example, collection of L-lysine can be usually performed by a combination of conventional techniques, for example, a method utilizing ion exchange resin, crystallization and others. Further, collection of L-glutamic acid can also be performed in a conventional manner, and it can be performed by, for example, a method utilizing ion exchange resin, crystallization or the like. Specifically, L-glutamic acid can be adsorbed on an anion exchange resin and isolated from it, or crystallized by neutralization. When both of L-lysine and L-glutamic acid are produced and used as a mixture, it is unnecessary to separate these amino acids from each other.

### Best Mode for Carrying out the Invention

Hereafter, the present invention will be more specifically explained with reference to the following examples.

### Example 1: Construction of coryneform bacterium introduced with fruA gene

### <1> Cloning of fruA gene of Escherichia coli JM109 strain

The nucleotide sequence of the *fruA* gene of *Escherichia coli* had already been elucidated (Genbank/EMBL/DDBJ accession No. M23196). The primers shown in Sequence Listing as SEQ ID NOS: 1 and 2 were synthesized based on the reported nucleotide sequence, and the fructose phosphotransferase gene was amplified by PCR utilizing chromosome DNA of *Escherichia coli* JM109 strain as a template.

Among the synthesized primers, that of SEQ ID NO: 1 corresponded to the sequence of from the 1st to the 24th nucleotides of the nucleotide sequence of the *fruA* gene of Genbank/EMBL/DDBJ accession No. M23196, and that of SEQ ID NO: 2 corresponded to the sequence of from the 2000th to the 1977th nucleotides of the same.

The chromosome DNA of *Escherichia coli* JM109 strain was prepared by a conventional method (Text for Bioengineering Experiments, Edited by the Society for Bioscience and Bioengineering, Japan, pp.97-98, Baifukan, 1992). Further, for PCR, the standard reaction conditions described in "Forefront of PCR", p.185 (compiled by Takeo Sekiya et al., Kyoritsu Shuppan, 1989).

The produced PCR product was purified in a conventional manner, then ligated to a plasmid pHC4 digested with SmaI by using a ligation kit (Takara Shuzo) and used for transformation of competent cells of *Escherichia coli* JM109 (Takara Shuzo). The cells were plated on L medium (10 g/L of Bacto trypton, 5 g/L of Bacto yeast extract, 5 g/L of NaCl, 15 g/L of agar, pH 7.2) containing 30 µg/ml of chloramphenicol and cultured overnight. Then, the emerged white colonies were picked up and separated into single colonies to obtain transformant strains. Plasmids were extracted from the obtained transformants, and a plasmid pHC4fru comprising the *fruA* gene ligated to the vector was obtained.

*Escherichia coli* harboring pHC4 was given with a private number of AJ12617 and deposited at the National Institute of Bioscience and Human-Technology, Agency of Industrial Science and Technology, Ministry of International Trade and Industry (1-3 Higashi 1-Chome, Tsukuba-shi, Ibaraki-ken, Japan, postal code: 305-8566) on April 24, 1991 and given with an accession number of FERM P-12215. Then, it was transferred to an international deposit under the provisions of the Budapest Treaty based on August 26, 1991 and given with an accession number of FERM BP-3532.

Then, in order confirm that the cloned DNA fragment coded for a protein having the fructose phosphotransferase activity, fructose phosphotransferase activity of the JM109 strain and the JM109 strain harboring pHC4fru was measured by the method described in Mori, M. & Shiio, I., Agric. Biol. Chem., 51, 129-138 (1987). As a result, it was confirmed that the JM109 strain harboring pHC4fru showed about 11 times higher fructose phosphotransferase activity compared with the JM109 strain not harboring pHC4fru, and thus it was confirmed that the *fruA* gene was expressed.

### <2> Introduction of pHC4fru into L-glutamic acid producing strain of coryneform bacterium and production of L-glutamic acid

The *Brevibacterium lactofermentum* AJ13029 strain was transformed with the plasmid pHC4fru by the electric pulse method (refer to Japanese Patent Laid-open Publication No. 2-207791) to obtain a transformant strain. Culture for L-glutamic acid production was performed as follows by using the obtained transformant strain AJ13029/pHC4fru. Cells of the AJ13029/pHC4fru strain obtained after culture on CM2B plate medium containing 5 µg/ml of chloramphenicol were inoculated into an L-glutamic acid production medium having the following composition containing 5 µg/ml of chloramphenicol and cultured at 31.5°C with shaking until the sugar in the medium was consumed. The obtained culture was inoculated into a medium having the same composition in 5% amount and cultured at 37°C with shaking until the sugar in the medium was consumed. As a control, the *Corynebacterium* bacterium AJ13029 strain transformed with the previously obtained plasmid pHC4 autonomously replicable in *Corynebacterium* bacteria by the electric pulse method was cultured in the same manner as described above.

### [L-Glutamic acid production medium]

The following components are dissolved (in 1 L), adjusted to pH 8.0 with KOH and sterilized at 115°C for 15 minutes.

| | |
|---|---|
| Fructose | 150 g |
| KH₂PO₄ | 2 g |
| MgSO₄·7H₂O | 1.5 g |
| FeSO₄·7H₂O | 15 mg |
| MnSO₄·4H₂O | 15 mg |
| Soybean protein hydrolyzed solution | 50 mL |
| Biotin | 2 mg |
| Thiamin hydrochloride | 3 mg |

After completion of the culture, the amount of L-glutamic acid accumulated in the culture broth was measured with Biotech Analyzer AS-210 produced by Asahi Chemical Industry Co., Ltd. The results are shown in Table 1.

**Table 1**

| Strain | Produced amount of L-glutamic acid (g/L) |
|---|---|
| AJ13029/pHC4 | 18.5 |
| AJ13029/PHC4fru | 20.5 |

### <3> Introduction of pHC4fru into L-lysine producing strain of coryneform bacterium and production of L-lysine

The *Brevibacterium lactofermentum* AJ11082 strain was transformed with the plasmid pHC4fru by the electric pulse method (refer to Japanese Patent Laid-open Publication No. 2-207791) to obtain a transformant strain. Culture for L-lysine production was performed as follows by using the obtained transformant strain AJ11082/pHC4fru. Cells of the AJ11082/pHC4fru strain obtained after culture on CM2B plate medium containing 5 µg/ml of chloramphenicol were inoculated into an L-lysine production medium having the following composition containing 5 µg/ml of chloramphenicol and cultured at 31.5°C with shaking until the sugar in the medium was consumed. As a control, the *Corynebacterium* bacterium AJ11082 strain transformed with the previously obtained plasmid pHC4 autonomously replicable in *Corynebacterium* bacteria by the electric pulse method was cultured in the same manner as described above.

The *Brevibacterium lactofermentum* AJ11082 was deposited at the Agricultural Research Service Culture Collection (1815 N. University Street, Peoria, Illinois 61604 U.S.A.) as an international deposit on January 31, 1981 and given with an accession number of NRRL B-11470.

### [L-Lysine production medium]

The following components are dissolved (in 1 L), adjusted to pH 8.0 with KOH, sterilized at 115°C for 15 minutes, and then added with calcium carbonate separately subjected to dry sterilization.

| | |
|---|---|
| Fructose | 100 g |
| (UH₄)₂SO₄ | 55 g |
| KH₂PO₄ | 1 g |
| MgSO₄·7H₂O | 1 g |
| Biotin | 500 µg |
| Thiamine | 2000 µg |
| FeSO₄·7H₂O | 0.01 g |
| MnSO₄·4H₂O | 0.01 g |
| Nicotinamide | 5 mg |
| Protein hydrolysate (soybean milk) | 30 mL |
| Calcium carbonate | 50 g |

After completion of the culture, the amount of L-lysine accumulated in the culture broth was measured with Biotech Analyzer AS-210 produced by Asahi Chemical Industry Co., Ltd. The results are shown in Table 2.

**Table 2**

| Strain | Produced amount of L-lysine (g/L) |
|---|---|
| AJ11082/pHC4 | 24.9 |
| AJ11082/PHC4fru | 28.4 |

### <4> Introduction of pHC4fru into L-lysine and L-glutamic acid producing strain of coryneform bacterium and simultaneous production of L-lysine and L-glutamic acid

The *Brevibacterium lactofermentum* AJ12993 strain was transformed with the plasmid pHC4fru by the electric pulse method (refer to Japanese Patent Laid-open Publication No. 2-207791) to obtain a transformant strain. Culture for L-lysine and L-glutamic acid production was performed as follows by using the obtained transformant strain AJ12993/pHC4fru. Cells of the AJ12993/pHC4fru strain obtained after culture on CM2B plate medium containing 5 µg/ml of chloramphenicol were inoculated into the aforementioned L-lysine production medium containing 5 µg/ml of chloramphenicol and cultured at 31.5°C. After 12 hours from the start of the culture, the culture temperature was shifted to 34°C, and the culture was further continued with shaking until the sugar in the medium was consumed. As a control, the *Corynebacterium* bacterium AJ12993 strain transformed with the previously obtained plasmid pHC4 autonomously replicable in *Corynebacterium* bacteria by the electric pulse method was cultured in the same manner as described above.

After completion of the culture, the amounts of L-lysine and L-glutamic acid accumulated in the culture broth was measured with Biotech Analyzer AS-210 produced by Asahi Chemical Industry Co., Ltd. The results are shown in Table 3.

**Table 3**

| Strain | Produced amount of L-lysine (g/L) | Produced amount of L-glutamic acid (g/L) |
|---|---|---|
| AJ12993/pHC4 | 8.5 | 18.5 |
| AJ12993/PHC4fru | 9.7 | 20.3 |

### Example 2: Isolation of fruA gene of Brevibacterium lactofermentum

### <1> Acquisition of fruA gene partial fragment of Brevibacterium lactofermentum ATCC13869

A region showing high homology for amino acid sequence in FruA among those of *Bacillus subtilis, Escherichia coli, Mycoplasma genitalium* and *Xanthomonas compestris* was selected, a nucleotide sequence was deduced from the amino acid sequence of that region, and the oligonucleotides shown as SEQ ID NOS: 3 and 4 were synthesized. Separately, chromosomal DNA of the *Brevibacterium lactofermentum* ATCC13869 strain was prepared by using Bacterial Genome DNA Purification Kit (Advanced Genetic Technologies Corp.). Sterilized water was added to 0.5 µg of the chromosomal DNA, 20 pmol each of the oligonucleotides, 4 µl of dNTP mixture (dATP, dGTP, dCTP, dTTP, 2.5 mM each), 5 µl of 10 x ExTaq Buffer (Takara Shuzo) and 1 U of ExTaq (Takara Shuzo) to prepare a PCR reaction mixture in a total volume of 50 µl. For this reaction mixture, PCR was performed for 25 cycles each consisting of denaturation at 98°C for 10 seconds, annealing at 45°C for 30 seconds and extension at 72°C for 90 seconds by using Thermal Cycler TP 240 (Takara Shuzo), and the PCR product was subjected to agarose gel electrophoresis. As a result, it was found that the reaction mixture contained an about 1.2 kb band.

The reaction product was ligated to pCR2.1 (Invitrogen) by using Original TA Cloning Kit (Invitrogen). After the ligation, competent cells of *Escherichia coli* JM109 (Takara Shuzo) were transformed with the ligation mixture, then plated on L medium (10 g/L of Bacto Trypton, 5 g/L of Bacto Yeast Extract, 5 g/L of NaCl 15 g/L of agar, pH 7.2) containing 10 µg/ml of IPTG (isopropyl-β-D-thiogalactopyranoside), 40 µg/ml of X-Gal (5-bromo-4-chloro-3-indolyl-β-D-galactoside) and 25 µg/ml of kanamycin, and cultured overnight. Then, the emerged white colonies were picked up and separated into single colonies to obtain transformant strains.

Plasmids were prepared from the obtained transformant strains by using the alkaline method (Text for Bioengineering Experiments, Edited by the Society for Bioscience and Bioengineering, Japan, p.105, Baifukan, 1992), and nucleotide sequences of the both ends of the inserted fragment were determined by the method of Sanger (J. Mol. Biol., 143, 161 (1980)) using the oligonucleotides shown as SEQ ID NOS: 5 and 6. Specifically, Big Dye Terminator Sequencing Kit (Applied Biosystems) was used for the nucleotide sequence determination, and analysis was performed by using Genetic Analyzer ABI 310 (Applied Biosystems). The determined nucleotide sequence was translated into an amino acid sequence, and it was compared with the amino acid sequences deduced from *fruA* genes of *Bacillus subtilis, Escherichia coli, Mycoplasma genitalium* and *Xanthomonas compestris*. As a result, it showed high homology, and thus the cloned fragment was determined to be the *fruA* gene derived from *Brevibacterium lactofermentum.*

### <2> Determination of whole nucleotide sequence of fruA gene of Brevibacterium lactofermentum ATCC13869

The fragment contained in the plasmid prepared in the above <1> was a partial fragment of the *fruA* gene, and thus it was further necessary to determine the nucleotide sequence of the *fruA* gene in full length. While there were inverse PCR (Genetics, 120, 621-623 (1988), a method utilizing LA-PCR In Vitro Cloning Kit (Takara Shuzo) and so forth as methods for determining an unknown nucleotide sequence flanking to a known region, the unknown sequence was determined by using LA-PCR In Vitro Cloning Kit in this example. Specifically, the oligonucleotides shown as SEQ ID NOS: 7, 8, 9 and 10 were synthesized based on the nucleotide sequence determined in the above <1>, and the determination was performed according to the protocol of LA-PCR In Vitro Cloning Kit.

For the 3' unknown region of the *fruA* gene partial fragment, chromosome DNA of the *Brevibacterium lactofermentum* ATCC13869 strain was treated with *Hind*III, ligated to *Hind*III Adapter contained in the kit and then used to perform PCR using the oligonucleotides of SEQ ID NOS: 7 and 11 as the primary PCR and PCR using the oligonucleotides of SEQ ID NOS: 8 and 12 as the secondary PCR. When this PCR product was subjected to agarose gel electrophoresis, a band of about 700 bp was observed. This band was purified by using Suprec ver. 2 (Takara Shuzo), and the nucleotide sequence of *fruA* gene contained in the 700 bp PCR product was determined by using the oligonucleotides of SEQ ID NOS: 8 and 12 in the same manner as described in <1>.

For the 5' unknown region of the *fruA* gene partial fragment, chromosome DNA of the *Brevibacterium lactofermentum* ATCC13869 strain was treated with *Bam*HI, ligated to *Sau3*AI Adapter contained in the kit and then used to perform PCR using the oligonucleotides of SEQ ID NOS: 9 and 11 as the primary PCR and PCR using the oligonucleotides of SEQ ID NOS: 10 and 12 as the secondary PCR. When this PCR product was subjected to agarose gel electrophoresis, a band of about 1500 bp was observed. This band was purified by using Suprec ver. 2 (Takara Shuzo), and the nucleotide sequence of *fruA* gene contained in the 1500 bp PCR product was determined by using the oligonucleotides of SEQ ID NOS: 10 and 12 in the same manner as described in <1>.

As for the nucleotide sequence determined as described above, the nucleotide sequence of about 3380 bp containing the *fruA* gene is shown in Sequence Listing as SEQ ID NO: 13. An amino acid sequence obtained by translating an open reading frame deduced from the above nucleotide sequence is shown as SEQ ID NO: 14. That is, a protein consisting of the amino acid sequence shown in Sequence Listing as SEQ ID NO: 14 is FruA of the *Brevibacterium lactofermentum* ATCC13869 strain. In addition, it is well known that a methionine residue at the N-terminus of a protein originates in ATG as a start codon and hence it does not relate to proper functions of the protein and removed by an action of peptidase after the translation in many cases. Removal of such a methionine residue might occur also in the aforementioned protein.

The above nucleotide sequence and amino acid sequence were compared with known sequences for homology. The used databases were GeneBank and SWISS-PROT. As a result, it was found that the DNA shown in Sequence Listing as SEQ ID NO: 13 was a novel gene in *Corynebacterium* bacteria showing homology with the already reported *fruA* genes.

The DNA shown as SEQ ID NO: 13 showed homology of 42.1%, 51.0%, 37.4% and 45.5% to *fruA* of *Bacillus subtilis, Escherichia coli, Mycobacterium genetilium* and *Xanthomonas compestris*, respectively, as the encoded amino acid. The nucleotide sequence and the amino acid sequence were analyzed by using Genetyx-Mac computer program (Software Development, Tokyo). The homology analysis was performed according to the method of Lipman and Peason (Science, 227, 1435-1441, 1985).

### Industrial Applicability

According to the present invention, production ability of coryneform bacteria for L-amino acids such as L-lysine or L-glutamic acid can be improved. Further,

## Claims

1. A method for producing an L-amino acid, comprising the steps of culturing a coryneform bacterium having enhanced intracellular fructose phosphotransferase activity and an ability to produce an L-amino acid in a medium to produce and accumulate the L-amino acid in the culture and collecting the L-amino acid from the culture wherein the fructose phosphotransferase activity is enhanced by increasing copy number of a gene coding for fructose phosphotransferase in a cell of the bacterium or by replacing an expression regulatory sequence of the gene.

2. The method according to claim 1, wherein the gene coding for fructose phosphotransferase is derived from an Escherichia bacterium.

3. The method according to claim 1, wherein the gene coding for fructose phosphotransferase is derived from a coryneform bacterium.

4. The method according to any one of claims 1 to 3, wherein the L-amino acid is selected from L-lysine, L-glutamic acid, L-threonine, L-isoleucine and L-serine.

5. The method according to claim 4, wherein the L-amino acid is selected from L-lysine and L-glutamic acid.

6. The method according to any one of claims 1 to 5, wherein the medium contains fructose as a carbon source.

## Patentansprüche

1. Verfahren zum Produktion einer L-Aminosäure, welches die Stufen umfasst, bei denen ein coryneformes Bakterium mit erhöhter intrazellulärer Fructosephosphatransferase-Aktivität und der Fähigkeit zur Produktion einer L-Aminosäure zur Produktion und Anhäufung der L-Aminosäure in der Kultur in einem Medium kultiviert und die L-Aminosäure aus der Kultur gewonnen wird, wobei die Fructosephosphatransferase-Aktivität durch Erhöhen der Kopienzahl eines für Fructosephosphatransferase kodierenden Gens in einer Zelle des Bakteriums oder durch Ersetzen der Expressionsregulationssequenz des Gens erhöht wird.

2. Verfahren nach Anspruch 1, wobei das für Fructosephosphatransferase kodierende Gen von einem Escherichia-Bakterium abgeleitet ist.

3. Verfahren nach Anspruch 1, wobei das für Fructosephosphatransferase kodierende Gen von einem coryneformen Bakterium abgeleitet ist.

4. Verfahren nach einem der Ansprüche 1 bis 3, wobei die L-Aminosäure unter L-Lysin, L-Glutaminsäure, L-Threonin, L-Isoleucin und L-Serin ausgewählt ist.

5. Verfahren nach Anspruch 4, wobei die L-Aminosäure unter L-Lysin und L-Glutaminsäure ausgewählt ist.

6. Verfahren nach einem der Ansprüche 1 bis 5, wobei das Medium Fructose als Kohlenstoffquelle enthält.

## Revendications

1. Procédé pour produire un L-animo acide, comprenant les étapes de culture d'une bactérie corynéforme ayant une activité fructose phosphotransférase intracellulaire accrue et une aptitude à produire L-animo acide dans un milieu pour produire et accumuler le L-animo acide dans la culture et de collecte du L-animo acide depuis la culture où l'activité frutose phosphotransférase est accru par l'augmentation du nombre de copie d'un gène codant la fructose phosphotransférase dans une cellule de la bactérie ou par remplacement d'une séquence régulant l'expression du gène.

2. Procédé selon la revendication 1 où le gène codant de la fructose phosphotransférase est dérivé d'une bactérie Escherichia.

3. Procédé selon la revendication 1 où le gène codant la fructose phosphotransférase est dérivé d'une bactérie corynéforme.

4. Procédé selon l'une quelconque des revendications 1 à 3 où le L-animo acide est choisi parmi le grand L-lysine, L-glutamique, L-thréonine, L-isoleucine et le L-sérine.

5. Procédé selon la revendication 4 où le L-animo acide est choisi parmi le L-lysine et l'acide L-glutamique.

6. Procédé selon l'une quelconque des revendications 1 à 5 où le milieu contient du fructose comme source de carbone.
